(19) Europäisches Patentamt
European Patent Office
Office européen des brevets

(11) Veröffentlichungsnummer: **0 097 791**
**B1**

(12) # EUROPÄISCHE PATENTSCHRIFT

(45) Veröffentlichungstag der Patentschrift: **13.03.85**

(51) Int. Cl.[4]: **C 07 C149/20**, C 07 C149/237, C 07 C148/00

(21) Anmeldenummer: **83104310.4**

(22) Anmeldetag: **02.05.83**

(54) **Verfahren zur Herstellung von beta-Mercaptopropionsäurederivaten.**

(30) Priorität: **26.06.82 DE 3223973**

(43) Veröffentlichungstag der Anmeldung: **11.01.84 Patentblatt 84/02**

(45) Bekanntmachung des Hinweises auf die Patenterteilung: **13.03.85 Patentblatt 85/11**

(84) Benannte Vertragsstaaten: **BE DE FR GB IT NL**

(56) Entgegenhaltungen:
**DE-B- 1 237 563**
**DE-B- 1 238 462**
**DE-B- 2 244 234**

(73) Patentinhaber: **Degussa Aktiengesellschaft**
**Weissfrauenstrasse 9**
**D-6000 Frankfurt am Main 1 (DE)**

(72) Erfinder: **Klenk, Herbert, Dr., Dipl.-Chem.**
**Grünaustrasse 19**
**D-6450 Hanau 9 (DE)**

**EP 0 097 791 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die Erfindung betrifft ein neues Herstellungsverfahren für β-Mercaptopropionsäurederivate der allgemeinen Formel (I),

$$H—S—CH_2—CH_2—X \qquad (I)$$

in der X für —CN, —COOH oder vorzugsweise für —COOR steht.

Es ist bekannt, β-Mercaptopropionsäurederivate durch Umsetzung von β-Chlorpropionsäurederivaten mit Alkalihydrogensulfid oder mit Thioharnstoff herzustellen. Diese Synthesen sind jedoch technisch nicht zufriedenstellend, weil sie sehr zeitaufwendig ablaufen oder nur schlechte Ausbeuten ergeben. (E. Reich, Organic chemistry of Bivalent Sulfur, Volume I, p. 431).

Es ist weiter bekannt, Acrylnitril mit Schwefelwasserstoff zu β-Mercaptopropionitril umzusetzen. Hierbei müssen aber erhebliche Überschüsse an Schwefelwasserstoff angewendet werden, wenn man Ausbeuten von etwa 38 % erzielen will. Außerdem ist es erforderlich, diese Umsetzung unter Druck vorzunehmen (US PS 2. 748. 155).

Auch bei Durchführung dieses Verfahrens in Gegenwart eines Katalysatorsystems, bestehend aus einer Base und Schwefel, konnten die grundsätzlichen Nachteile dieses Verfahrens nicht beseitigt werden (DE-OS 2 034 172).

Schließlich ist noch bekannt, daß eine direkte Addition von Schwefelwasserstoff an Acrylester zwecks Bildung der Mercaptopropionsäureester nicht möglich ist, da stets die Thiodipropionate erhalten werden (Riddel, Monomeric Acrylic Esters, 1954, Seite 148). Um solche Doppeladditionen zu vermeiden, ist es bekannt geworden, Acrylsäureester mit Alkalihydrogensulfiden unter Verwendung von Schwefelkohlenstoff als Reaktionsmedium umzesetzen. Bei dieser Umsetzung muß aber ein organisches Lösungsmittel, beispielsweise ein Alkohol, mitverwendet werden. Es wird dabei darauf hingewiesen, daß im Reaktionsgemisch möglichst kein Wasser vorhanden sein soll, da sonst die Bildung von Thiodipropionsäureestern bevorzugt ablaüft. Im technischen Maßstab können jedoch diese wasserfreien Bedingungen nur schwer realisiert werden. Außerdem wird die Aufarbeitung durch ein zusätzliches Lösungsmittel erschwert, da es in der Regel die Isolierung des entstandenen Mercaptoderivates aus der angesäuerten, wäßrigen Reaktionsmischung behindert, und zudem eine destillative Trennung von Lösungsmittel und Schwefelkohlenstoff erforderlich macht (DE-PS 2 244 234).

Es wurde nun gefunden, daß man β-Mercaptopropionsäurederivate der allgemeinen Formel (I),

$$H—S—CH_2—CH_2—X \qquad (I),$$

in der X für —CN, —COOH oder vorzugsweise für —COOR steht, und wobei R einen Alkylrest mit 1 bis 18, insbesondere mit 1 bis 8 Kohlenstoffatomen, vorzugsweise den Methylrest, bedeutet, in hohen Ausbeuten herstellen kann, wenn man ein Acrylsäurederivat der allgemeinen Formel (II),

$$CH_2 = CH—X \qquad (II),$$

in der X die oben angegebenen Bedeutungen hat, mit einem Erdalkali-, Alkali- oder Ammoniumsalz der Trithiocarbonsäure in einem Gemisch mit Wasser und einem organischen Lösungsmittel oder vorzugsweise in Wasser umsetzt, und das erhaltene Reaktionsgemisch nach Ansäuern mit einer Protonensäure in an sich bekannter Weise aufarbeitet.

Die für die Umsetzung benötigten Alkali- oder Erdalkalisalze der Trithiocarbonsäure können in einfacher Weise aus Schwefelkohlenstoff und den entsprechenden Sulfiden in wassriger Lösung gewonnen werden. Diese Trithiocarbonate entsprechen beispielsweise folgenden Formeln :

$$Na_2CS_3,\ K_2CS_3,\ CaCS_3,\ (NH_4)_2CS_3.$$

Ein besonderer Vorteil des erfindungsgemäßen Verfahrens ist darin zu sehen, daß die Umsetzung in Wasser durchgeführt werden kann. Es ist jedoch auch möglich, Gemische von Wasser mit wasserlöslichen oder wasserunlöslichen organischen Lösungsmitteln auzuwenden.

Beispiele für solche Lösungsmittel sind : Alkohole wie Methanol, Ethanol, Butanol oder Glykol. Es kann beispielsweise aber auch Aceton, Dioxan, Benzol oder Methylenchlorid verwendet werden.

Die erfindungsgemäße Umsetzung verläuft sehr rasch. Die Anwendung sehr hoher Temperaturen oder hoher Drucke ist daher nicht erforderlich. Das Vorliegen salzhaltiger Wasserlösungen erlaubt auch das Arbeiten unterhalb 0 °C. Im allgemeinen können Reaktionstemperaturen zwischen etwa − 25 °C und 80 °C eingehalten werden. Vorzugsweise werden die Umsetzungen in dem Temperaturbereich zwischen − 10 °C und 40 °C durchgeführt. Die Reaktion kann kontinuierlich oder diskontinuierlich durchgeführt werden.

Die Umsetzung kann unter Einhaltung eines Molverhätnisses von Acrylsäurederivat (II) zum Salz der Trithiocarbonsäure von 1 : 2 durchgeführt werden. Auch ein kleineres Verhältnis ist möglich, bringt

jedoch keine weiteren Vorteile. Im allgemeinen hält man ein Verhältnis von 1 : 1-1,5, vorzugsweise von 1 : 1-1,2, ein. Es können auch unterschüssige Mengen des Trithiocarbonatsalzes eingesetzt werden, jedoch ergibt dies im allgemeinen keine Vorteile.

Während der Umsetzung von Trithiocarbonat mit dem Acrylsäurederivat ist in der Reaktionsmischung in der Regel kein freier Schwefelkohlenstoff nachweisbar. Zur Freisetzung der Mercaptopropionsäurederivates und des Schwefelkohlenstoffes werden nach dem erfindungsgemäßen Verfahren insgesamt mindestens 2 Äquivalente Säure pro Mol eingesetztem Trithiocarbonat benötigt. Es können auch weniger als 2 Äquivalente verwendet werden, doch bringt dies in der Regel keine Vorteile.

Die Zugabe dieser 2 Äquivalente Säure kann nach dem Reaktionsende erfolgen. Im Hinblick auf die Alkaliempfindlichkeit bestimmter Acrylsäureester kann es jedoch empfehlenswert sein, eine zum Acrylester äquivalente Menge der Säure gleichlaufend der Umsetzung der beiden Komponenten zuzugeben. Als Saüre kann eine jede Protonensäure eingesetzt werden. Besonders geeignet sind aber wässrige Säuren wie Phosphorsäure, Schwefelsäure oder insbesondere Salzsäure.

Die Aufarbeitung des Reaktionsgemisches erfolgt in an sich bekannter Weise. Beispielsweise wird das zweiphasige Gemisch aufgetrennt und die organische Phase fraktioniert destilliert. Hierbei kann der Schwefelkohlenstoff isoliert und gegebenenfalls zur Bildung der Trithiocarbonate wieder verwendet werden.

Die nach dem erfindungsgemäßen Verfahren herstellbaren β-Mercaptopropionsäurederivate sind wertvolle Zwischenprodukte beispielsweise für die Herstellung von Pestiziden und für die Herstellung von Lackstabilisatoren. Sie können aber auch als Kosmetikaprodukte bei der Herstellung von Kalt- und Dauerwellenpräparaten verwendet werden.

Beispiel 1

In einer Rührapparatur werden 376 g einer 45 %igen Natriumtrithiocarbonatlösung (entsprechend 1,1 Mol) vorgelegt. Unter Rühren werden bei 1 °C gleichzeitig 86 g Acrylsäuremethylester (1,0 Mol) und 100 ml einer 32 %igen Salzsaüre (1,0 Mol) zugetropft. Nach Zutropfen wird noch 10 Minuten nachgerührt und dann unter weiteren Rühren 150 ml der 32 %igen salzsäure zugegeben. das gebildete zweiphasige Gemisch wird in einen Scheidetrichter überführt, die organische Phase abgetrennt und fraktioniert destilliert. Es werden 110,4 g β-Mercaptopropionsäuremethylester vom Siedepunkt 60 °C bei 18 mbar isoliert. Dies enstpricht einer Ausbeute von 92 % bezogen auf eingesetzten Acrylester.

Es wurden außerdem 76 g Schwefelkohlenstoff zurückgewonnen.

Beispiel 2

Es wird, wie in Beispiel 1 beschrieben, verfahren, jedoch werden anstelle von Acrylsäuremethylester 128 g (1,0 Mol) Butylacrylat eingesetzt, und es wird bei + 20 °C gearbeitet. Nach de fraktionierten Destillation werden 136 g (entspricht einer Ausbente von 84 %) β-Mercaptopropionsäurebutylester vom Siedepunkt 102 °C bei 18 mbar isoliert. Außerdem werden 75 g Schwefelkohlenstoff zurückgewonnen.

Beispiel 3

Es wird, wie in Beispiel 1 beschrieben, verfahren, jedoch anstatt Acrylsäuremethylester 53 g (1,0 Mol) Acrylnitril eingesetzt. Nach dem Ansäuern mit Salzsäure und der Phasentrennung wird noch einmal mit Ether ausgeschüttelt : Die vereinigten Phasen ($CS_2$- und Etherphase) werden dann fraktioniert destilliert. Es werden 62 g β-Mercaptonitril mit einem Siedepunkt von 82 °C bei 20 mbar erhalten. Das entspricht einer Ausbeute von 71 % bezogen auf eingesetzes Acrylnitril.

Beispiel 4

Es wird, wie in Beispiel 1 beschrieben, verfahren, jedoch werden anstelle von Acrylsäuremethylester 184 g Acrylsäureoctylester eingesetzt und zunächst 250 ml verdünnte $H_2SO_4$(4 N), und dann später 300 ml verdünnte $H_2SO_4$ zugegeben. Nach der fraktionierten Destillation werden 174,5 g (entspricht einer Ausbeute von 80 %) 3-Mercaptopropionsäureoctylester isoliert.

Beispiel 5

Es wird, wie in Beispiel 1 beschrieben, verfahren, jedoch werden anstelle von Acrylsäuremethylester 352 g eines 92 %igen Acrylsäureoctadecylesters (1,0 Mol) eingesetzt. Nach dem Ansäuern wird die gesamte Reaktionsmischung filtriert, wobei 350 g eines weißen Feststoffes zurückbleiben, der nach dem Trocknen oberhalb 40 °C zu schmelzen beginnt. Eine Elementaranalyse zeigt einen S-H-gehalt von 8,3 %.

Beispiel 6

In einer Rührapparatur werden 577,5 g einer 40 %igen Natriumtrithiocarbonatlösung (entsprechend

1,5 Mol) vorgelegt. Unter Rühren werden bei −10 °C 128 g Acrylsäureisobutylester (1,0 Mol) zugetropft. Anschliessend wird noch 10 Minuten nachgerührt und dann mit 350 ml 32 %iger Salzsäure angesäuert. Nach der Phasentrennung wird die organische Phase fraktioniert destilliert. Es werden 128 g Mercaptopropionsäureisobutylester (entspricht einer Ausbeute von 79 %) erhalten.

Beispiel 7

Es wird, wie in Beispiel 6 beschrieben, verfahren, jedoch werden statt Acrylsäureisobutylester 53 g (1,0 Mol) Acrylnitril eingesetzt. Es werden nach Extraktion der Reaktionsmischung mit Ether und anschließender fraktionierter Destillation 63,5 g (73 % Ausbeute) β-Mercaptopropionitril erhalten.

Beispiel 8

In einem kleinen Reaktionskolben von 40 ml Inhalt, der einen Überlauf besitzt und mit einem Magnetrührere gerührt wird, werden über zwei Pumpen getrennt stündlich eine Menge von 627 g einer 54,1 %igen Natriumtrithiocarbonatlösung (≙ 2,2 Mol/h) und 79,2 g Acrylsäure (≙ 1,1 Mol/h) zudosiert. Durch Außenkühlung wird in dem Reaktor eine Temperatur unter 20 °C eingehalten. Die aus dem Reaktionskolben überlaufende Lösung gelangt über eine Verweilzeitstrecke in einen zweiten Reaktionskolben von 35 ml Inhalt, der ebenfalls einen Überlauf sowie eine Entlüftung besitzt und in den pro Stunde 550 ml einer 25 %igen Salzsäure eindosiert werden. Die aus dem Reaktor überlaufende Emulsion wird chargenweise aufgefangen, das enstandene zweiphasige Gemisch getrennt, die wässrige Phase mit Ether extrahiert und die gesammelten organischen Extrakte fraktioniert destilliert. So werden aus einer Charge, die während einer Stunde aus dem zweiten Reaktionskolben abläuft, 70,4 g β-Mercaptopropionsäure vom Siedepunkt 113-117 °C bei 18 mbar erhalten. Das entspricht einer Ausbeut 60,4 % bezogen auf Acrylsäure. Die erhaltene β-Mercaptopropionsäure erstarrt beim Abkühlen unter 16 °C.

**Ansprüche**

1. Verfahren zur Herstellung von β-Mercaptopropionsäuredirivaten der allgemeinen Formel (I),

$$H{-}S{-}CH_2{-}CH_2{-}X \qquad (I),$$

in der X für —CN, —COOH ode vorzugsweise für —COOR steht, und wobei R einen Alkylrest mit 1 bis 18, insbesondere mit 1 bis 8, Kohlenstoffatomen, vorzugsweise den Methylrest, bedeutet, dadurch gekennzeichnet, daß man ein Acrylsäurederivat der allgemeinen Formel (II),

$$CH_2 = CH{-}X \qquad (II),$$

in der X die oben angegebenen Bedeutungen hat, mit einem Erdalkali-, Alkali- oder Ammoniumsalz der Trithiocarbonsäure in einem Gemisch aus Wasser und einem organischen Lösungsmittel, oder vorzugsweise in Wasser umsetzt, und das erhaltene Reaktionsgemisch nach Ansäuern mit einer Protonensäure in an sich bekannter Weise aufarbeitet.

2. Verfahren nach Anspruch 1, dadurch gekennzeichnet, daß man bei einer Temperatur zwischen − 25 °C und 80 °C, vorzugsweise zwischen − 10 °C und 40 °C, umsetzt.

3. Verfahren nach den Ansprüchen 1 und 2, dadurch gekennzeichnet, daß man das Acrylsäurederivat (II) und das Salz der Trithiocarbonsäure im Mol-Verhältnis von 1 : 1-2, vorzugsweise von 1 : 1-1,2, einsetzt.

4. Verfahren nach den Ansprüchen 1 bis 3, dadurch gekennzeichnet, daß man im Maße des Fortschreitens der Umsetzung des Trithiocarbonates mit dem Acrylsäurederivat der Formel II in bezug auf letzteres bis zu einem Äquivalent einer Protonensäure zugibt.

**Claims**

1. A process for the production of β-mercaptopropionic acid derivatives corresponding to the general formula (I),

$$H{-}S{-}CH_2{-}CH_2{-}X \qquad (I),$$

wherein X represents —CN, —COOH or preferably —COOR, and R represents an alkyl radical having from 1 to 18 and particularly from 1 to 8 carbon atoms, preferably a methyl radical, characterized in that an acrylic acid derivative corresponding to the general formula (II)

$$CH^2 = CH{-}X,$$

wherein X is as defined above, is reacted in a mixture of water and an organic solvent or preferably in water with an alkaline earth metal, alkali metal or ammonium salt of trithiocarbonic acid and the resulting reaction mixture is worked up in known manner after acidifying with a protonic acid.

2. A process according to claim 1, characterised in that the reaction is carried out at a temperature of from −25 °C to 80 °C, preferably at a temperature of from −10 °C to 40 °C.

3. A process according to claims 1 and 2, characterised in that the acrylic acid derivative (II) and the salt of the trithiocarbonic acid are used in a mol ratio of 1 : 1-2, preferably of 1 : 1-1,2.

4. A process according to claims 1 to 3, characterised in that as the reaction of the trithiocarbonate with the acrylic acid derivative corresponding to formula (II) progresses, up to an equivalent of a protonic acid is added, based on the latter.

**Revendications**

1. Procédé pour la fabrication de dérivés de l'acide β-mercaptopropionique, répondant à la formule générale (I) :

$$H—S—CH_2—CH_2—X \quad (I)$$

où X représente —CN, —COOH, ou de préférence —COOR, R étant un radical alcoyle avec 1 à 18, en particulier 1 à 8 atomes de carbone, de préférence le radical méthyle, procédé caractérisé en ce que l'on fait réagir un dérivé de l'acide acrylique répondant à la formule générale (II) ;

$$CH_2 = CH—X \quad (II)$$

où X a les mêmes significations que ci-dessus, avec un sel alcalino-terreux, alcalin, ou ammoniacal de l'acide trithiocarbonique, dans un mélange d'eau et d'un solvant organique, ou de préférence dans l'eau, et que l'on continue le traitement du mélange réactionnel obtenu, après acidification avec un acide protonique, de la façon connnue.

2. Procédé suivant la revendication 1, caractérisé en ce que la réaction s'effectue à une température qui se situe entre -25 et 80 °C de préférence entre −10 °C et 40 °C.

3. Procédé suivant les revendications 1 et 2, caractérisé en ce que l'on met en oeuvre le dérivé de l'acide acrylique (II) et le sel de l'acide trithiocarbonique dans un rapport de 1 : 1 à 2 de préférence de 1 : 1 à 1,2.

4. Procédé suivant les revendications 1 à 3, caractérisé en ce que l'on ajoute jusqu'à un équivalent d'acide protonique dans la mesure de l'avancement de la réaction du trithiocarbonate avec le dérivé de l'acide acrylique de formule (II), en rapport avec ce dernier.